# EUROPEAN PATENT APPLICATION

(11) **EP 4 458 856 A1**
(43) Date of publication of application: **06.11.2024**
(21) Application number: 24172597.7
(22) Date of filing: 26.04.2024
(51) Int. Cl.: C07K 16/28, A61P 35/00

(54) **METHOD FOR PRODUCING ANTI-EGFR ANTIBODY, METHOD FOR IMPROVING AFFINITY FOR FC GAMMA RIIIA, AND ANTI-EGFR ANTIBODY**

(30) Priority: 27.04.2023 JP 2023073449
(71) Applicant: SYSMEX CORPORATION, Kobe-shi Hyogo 651-0073 (JP)
(72) Inventor: EGASHIRA, Yuriko, Kobe-shi, Hyogo, 651-0073 (JP); MAETA, Shingo, Kobe-shi, Hyogo, 651-0073 (JP)
(74) Representative: De Clercq & Partners

(57) **Abstract**

Disclosed is a method for producing an anti-EGFR antibody, including: generating an anti-EGFR antibody having an amino acid sequence of the anti-EGFR antibody using a polynucleotide encoding the amino acid sequence; and collecting the anti-EGFR antibody, wherein the anti-EGFR antibody contains an HCDR1 including the amino acid sequence of SEQ ID NO: 1, an HCDR2 including the amino acid sequence SGG, an HCDR3 including the amino acid sequence of SEQ ID NO: 2, an LCDR1 including the amino acid sequence of SEQ ID NO: 3, an LCDR2 including the amino acid sequence YAS, and an LCDR3 including the amino acid sequence of SEQ ID NO: 4.

## Description

### FIELD OF THE INVENTION

The present invention relates to a method for producing an anti-EGFR antibody. The present invention relates to a method for improving the affinity of an anti-EGFR antibody for FcyRIIIa. The present invention relates to an anti-EGFR antibody.

### BACKGROUND

Techniques for modifying the amino acid sequence of an antibody to change the property of the antibody are known. For example, U.S. Patent Application Publication No. 2013/0303406 describes that aggregation of an antibody is suppressed by modifying a framework region 2 (FR2) and a framework region 3 (FR3) of the antibody to enhance solubility and stability of the antibody. In addition, U.S. Patent Application Publication No. 2018/0179298 describes that affinity of an antibody is controlled by modifying FR3.

### SUMMARY OF THE INVENTION

An object of the present invention is to provide an antibody obtained by modifying an anti-EGFR antibody used in an anticancer agent or the like to improve the affinity for FcyRIIIa. In addition, an object of the present invention is to provide a method for producing the antibody and a method for improving the affinity of an anti-EGFR antibody for FcyRIIIa.

The present inventors have found that the affinity of an anti-EGFR antibody for FcyRIIIa can be improved by modifying at least three amino acid residues in the light chain FR3 of the anti-EGFR antibody to arginine residues or aspartic acid residues, and have completed the present invention.

The present invention provides the following inventions [1] to [17].
[1] A method for producing an anti-EGFR antibody, comprising: generating an anti-EGFR antibody having an amino acid sequence of the anti-EGFR antibody using a polynucleotide encoding the amino acid sequence; and collecting the anti-EGFR antibody,
   wherein the anti-EGFR antibody comprises: an HCDR1 comprising an amino acid sequence of SEQ ID NO: 1, an HCDR2 comprising an amino acid sequence SGG, an HCDR3 comprising an amino acid sequence of SEQ ID NO: 2, an LCDR1 comprising an amino acid sequence of SEQ ID NO: 3, an LCDR2 comprising an amino acid sequence YAS, and an LCDR3 comprising an amino acid sequence of SEQ ID NO: 4,
   the anti-EGFR antibody comprises an amino acid sequence in which at least three amino acid residues in a framework region 3 of a light chain have been modified each independently (from a residue which is other than an arginine or aspartic acid residue) to an arginine residue or aspartic acid residue, and
   the HCDR1, HCDR2, HCDR3, LCDR1, LCDR2, LCDR3, and the framework region 3 are defined by a Chothia method.
[2] A method for improving affinity of an anti-EGFR antibody for FcyRIIIa as compared with affinity before modification, comprising modifying at least three amino acid residues in a framework region 3 of a light chain each independently (from a residue which is other than an arginine or aspartic acid residue) to an arginine residue or aspartic acid residue in the anti-EGFR antibody,
   wherein the anti-EGFR antibody comprises an HCDR1 comprising an amino acid sequence of SEQ ID NO: 1, an HCDR2 comprising an amino acid sequence SGG, an HCDR3 comprising an amino acid sequence of SEQ ID NO: 2, an LCDR1 comprising an amino acid sequence of SEQ ID NO: 3, an LCDR2 comprising an amino acid sequence YAS, and an LCDR3 comprising an amino acid sequence of SEQ ID NO: 4, and
   the HCDR1, HCDR2, HCDR3, LCDR1, LCDR2, LCDR3, and the framework region 3 are defined by a Chothia method.
[3] The method according to [1] or [2], wherein the at least three amino acid residues are at least three amino acid residues selected from the group consisting of amino acid residues at positions 63, 65, 67, 70, and 72 of the light chain.
[4] The method according to any one of [1] to [3], wherein the at least three amino acid residues include amino acid residues at positions 65, 67, and 70 of the light chain.
[5] The method according to any one of [1] to [3], wherein the at least three amino acid residues include amino acid residues at positions 65, 67, 70, and 72 of the light chain.
[6] The method according to any one of [1] to [3], wherein the at least three amino acid residues include amino acid residues at positions 63, 65, 67, 70, and 72 of the light chain.
[7] The method according to any one of [1] to [6], wherein the heavy chain of the anti-EGFR antibody has the amino acid sequence of SEQ ID NO: 5.
[8] The method according to any one of [1] to [3], or any one of [7] not depending on [4] to [6], wherein the light chain of the anti-EGFR antibody contains the amino acid sequence of SEQ ID NO: 6, the amino acid sequence of SEQ ID NO: 7, or the amino acid sequence of SEQ ID NO: 8.
[9] The method according to any one of [1] to [8], wherein the at least three amino acid residues are 3 or more and 5 or less amino acid residues.
[10] An anti-EGFR antibody, comprising an HCDR1 comprising an amino acid sequence of SEQ ID NO: 1; an HCDR2 comprising an amino acid sequence SGG; an HCDR3 comprising an amino acid sequence of SEQ ID NO: 2; an LCDR1 comprising an amino acid sequence of SEQ ID NO: 3; an LCDR2 comprising an amino acid sequence YAS; and an LCDR3 comprising an amino acid sequence of SEQ ID NO: 4,
   wherein at least three amino acid residues in a framework region 3 of a light chain have been modified each independently to arginine residue or aspartic acid residue, and
   the HCDR1, HCDR2, HCDR3, LCDR1, LCDR2, LCDR3, and the framework region 3 are defined by a Chothia method.
[11] The antibody according to [10], wherein the at least three amino acid residues are at least three amino acid residues selected from the group consisting of amino acid residues at positions 63, 65, 67, 70, and 72 of the light chain.
[12] The antibody according to [10] or [11], wherein the at least three amino acid residues include amino acid residues at positions 65, 67, and 70 of the light chain.
[13] The antibody according to [10] or [11], wherein the at least three amino acid residues include amino acid residues at positions 65, 67, 70, and 72 of the light chain.
[14] The antibody according to [10] or [11], wherein the at least three amino acid residues include amino acid residues at positions 63, 65, 67, 70, and 72 of the light chain.
[15] The antibody according to any one of [10] to [14], wherein the heavy chain has the amino acid sequence of SEQ ID NO: 5.
[16] The antibody according to any one of [10] or [11], or any one of [15] not depending on [12] to [14], wherein the light chain contains the amino acid sequence of SEQ ID NO: 6, the amino acid sequence of SEQ ID NO: 7, or the amino acid sequence of SEQ ID NO: 8.
[17] The antibody according to any one of [10] to [16], wherein the at least three amino acid residues are 3 or more and 5 or less amino acid residues.

According to the present invention, there are provided an anti-EGFR antibody having improved affinity for FcyRIIIa, a method for producing the antibody, and a method for improving the affinity of an anti-EGFR antibody for FcyRIIIa.

### BRIEF DESCRIPTION OF THE DRAWINGS

Fig. 1 is a graph showing a result of an Example.
Fig. 2 is an enlarged view of a part of Fig. 1.

### DETAILED DESCRIPTION OF THE PREFERRED EMBODIMENTS

### (Anti-EGFR antibody)

The anti-EGFR antibody of the present inventions is characterized in that at least three amino acid residues in the FR3 of the light chain defined by the Chothia method have been modified from a residue which is other than an arginine or aspartic acid residue to arginine residues or aspartic acid residues. The anti-EGFR antibody having the amino acid sequence before the modification is referred to as "unmodified anti-EGFR antibody" or simply "unmodified antibody". The at least three amino acid residues before the modification are amino acid residues other than an arginine residue and an aspartic acid residue. Accordingly, an unmodified anti-EGFR antibody is preferably an antibody which does not comprise at least three arginine or aspartic acid residues in the FR3 of the light chains as determined by the Chothia method. In particular embodiments, an unmodified anti-EGFR antibody is preferably an antibody which does not comprise at least four arginine residues, at least five aspartic acid residues, or at least six residues which are either arginine or aspartic acid residues in the FR3 of the light chain as defined by the Chothia method. By the modification, the affinity of the antibody for FcyRIIIa is improved as compared with that before the modification. This improvement is expected to improve the antibody-dependent cellular cytotoxicity (ADCC) activity of the anti-EGFR antibody. As a result of the modification, the anti-EGFR antibody comprises at least three additional arginine residues or aspartic acid residues in the FR3 of the light chain defined by the Chothia method compared to the unmodified antibody. In particular embodiments, the EGFR antibody according to the invention comprises at least four arginine residues, at least six aspartic acid residues, or at least seven residues which are either arginine or aspartic acid residues in the FR3 of the light chain as defined by the Chothia method. In particular embodiments, the EGFR antibody according to the invention comprises at least four arginine residues or at least eight aspartic acid residues in the FR3 of the light chain as defined by the Chothia method. In particular embodiments, the EGFR antibody according to the invention comprises four arginine residues or five arginine residues. In particular embodiments, the EGFR antibody according to the invention comprises eight aspartic acid residues or five arginine residues. However, as will be understood preferably, the (modified) residues are either all arginine residues or all aspartic acid residues, preferably arginine residues.

The framework region (FR) is a region other than the complementarity determining region (CDR) present in the variable region of each of the light chain and the heavy chain of an antibody. Three CDRs are present in each variable region of the light chain and the heavy chain of an antibody to compose an antigen-binding site of the antibody. The three CDRs are referred to as CDR1, CDR2, and CDR3 from the N-terminus of an antibody. In the present specification, the CDR1, CDR2, and CDR3 of the heavy chain are referred to as HCDR1, HCDR2, and HCDR3, respectively, and the CDR1, CDR2, and CDR3 of the light chain are referred to as LCDR1, LCDR2, and LCDR3, respectively. The FR serves as a scaffold for linking three CDRs, thereby contributing to the structural stability of the CDRs. FR3, one of the FRs, refers to a region between the CDR2 and the CDR3.

As a method for numbering the amino acid sequence of an antibody, any known method may be used. For example, the Chothia method (Chothia C. and Lesk AM., Canonical Structures for the Hypervariable Regions of Immunoglobulins., J Mol Biol., vol. 196, p. 901-917, 1987), the Kabat method (Kabat EA. et al., Sequences of Proteins of Immunological Interest., NIH publication No. 91-3242), the IMGT method (Lefranc MP., IMGT Unique Numbering for the Variable (V), Constant (C), and Groove (G) Domains of IG, TR, MH, IgSF, and MhSF., Cold Spring Harb Protoc. 2011(6): 633-642, 2011), the Honergger method (Honegger A. et al., Yet Another Numbering Scheme for Immunoglobulin Variable Domains: An Automatic Modeling and Analysis Tool., J Mol Biol., vol. 309, p. 657-670, 2001), the ABM method, the Contact method, and the like are known. Herein, the numbering of the amino acid sequence of the anti-EGFR antibody is described according to the definition of the Chothia method. The CDR and FR herein are based on the Chothia method.

In the anti-EGFR antibody, at least three modifications may be introduced at any point in the light chain FR3. Preferably, the modification is introduced into a region from which an amino acid residue that is folded inside the molecule and is not exposed on the surface (hereinafter, also referred to as "unexposed residue") is excluded . In particular embodiments, the amino acid residue region excluding the unexposed residue from FR3 of the light chain is specifically the region corresponding to amino acid residues at positions 57 to 81 of the light chain.

More preferably, the modification is introduced into a region of the anti-EGFR antibody from which the unexposed residue and the Vernier zone residue are excluded. The Vernier zone residue is an amino acid residue contributing to the structural stability of CDR among amino acid residues contained in the FR. Therefore, it is preferred that no modification be introduced into the Vernier zone residue. The amino acid residues corresponding to a region from which both the unexposed residue and the Vernier zone residue are excluded from FR3 of the light chain are specifically amino acid residues at positions 57 to 63, 65, 67, 70, and 72 to 81 of the light chain. Thus in particular embodiments, the modifications are introduced in amino acid residues selected from those at positions 57 to 63, 65, 67, 70, and 72 to 81 of the light chain. Accordingly, in particular embodiments the anti-EGFR antibody of the invention comprises at least four arginine residues, at least five aspartic acid residues, or at least six residues which are either arginine or aspartic acid residues at positions 57 to 63, 65, 67, 70, and 72 to 81 of the light chain. In particular embodiments, the anti-EGFR antibody of the invention comprises at least five arginine residues or at least six aspartic acid residues at positions 57 to 63, 65, 67, 70, and 72 to 81 of the light chain.

More preferably, within the amino acid residue region from which the unexposed residue and the Vernier zone residue are excluded in the anti-EGFR antibody, the modification is introduced into an amino acid residue whose side chain faces the molecular surface. By modifying the amino acid residue whose side chain faces the molecular surface, the contribution to the surface charge becomes greater. The amino acid residue whose side chain in the FR3 of the light chain faces the molecular surface is specifically amino acid residues at positions 57, 60, 63, 65, 67, 70, 72, 74, 76, 77, and 79 to 81 of the light chain. Among them, the positions 63, 65, 67, 70, and 72 of the light chain are particularly preferable as candidates for the introduction site of the modification. In one example, the anti-EGFR antibody is modified at three amino acid residues at positions 65, 67 and 70 of the light chain. In another example, the anti-EGFR antibody is modified at four amino acid residues at positions 65, 67, 70, and 72 of the light chain. In yet another example, the anti-EGFR antibody is modified at five amino acid residues at positions 63, 65, 67, 70, and 72 of the light chain. Accordingly, in particular embodiments, the anti-EGFR antibody of the invention comprises an arginine or aspartic acid residue in at least three positions selected from positions 57, 60, 63, 65, 67, 70, 72, 74, 76, 77, and 79 to 81 of the light chain. More preferably, the anti-EGFR antibody of the invention comprises an arginine or aspartic acid residue in at least three positions selected from positions 63, 65, 67, 70, and 72 of the light chain. In a further embodiment the anti-EGFR antibody of the invention comprises an arginine or aspartic acid residue in at least three positions selected from positions 65, 67, 70, and 72 of the light chain. In a further embodiment the anti-EGFR antibody of the invention comprises an arginine or aspartic acid residue in at least three positions selected from positions 65, 67, and 70 of the light chain.

The number of modifications introduced into the FR3 is not particularly limited as long as the function of the anti-EGFR antibody is not significantly impaired, but is preferably 13 amino acids or less. That is, the number of modifications is specifically 3, 4, 5, 6, 7, 8, 9, 10, 11, 12, or 13. More preferably, the number of modifications is 5 or less.

The modification is a modification in which the amino acid residue other than an arginine residue and an aspartic acid residue at the predetermined position is changed to an arginine residue or an aspartic acid residue. For the modification of amino acid residue introduced into the anti-EGFR antibody, all modifications may be modifications to arginine residues, or all modifications may be modifications to aspartic acid residues. Some of the modification sites may be modified to an arginine residue, and other sites may be modified to an aspartic acid residue. That is, the modification may be at least three modifications of a modification to an arginine residue or an asparagine residue at position 63 of the light chain, a modification to an arginine residue or an asparagine residue at position 65 of the light chain, a modification to an arginine residue or an asparagine residue at position 67 of the light chain, a modification to an arginine residue or an asparagine residue at position 70 of the light chain, or a modification to an arginine residue or an asparagine residue at position 72 of the light chain. When three amino acid residues at positions 65, 67, and 70 of the light chain are modified, the position 65 of the light chain is modified to an arginine residue or an asparagine residue, the position 67 of the light chain is modified to an arginine residue or an asparagine residue, and the position 70 of the light chain is modified to an arginine residue or an asparagine residue. When the four amino acid residues at positions 65, 67, 70, and 72 of the light chain are modified, the position 65 of the light chain is modified to an arginine residue or an asparagine residue, the position 67 of the light chain is modified to an arginine residue or an asparagine residue, the position 70 of the light chain is modified to an arginine residue or an asparagine residue, and the position 72 of the light chain is modified to an arginine residue or an asparagine residue. When five amino acid residues at positions 63, 65, 67, 70, and 72 of the light chain are modified, the position 63 of the light chain is modified to an arginine residue or an asparagine residue, the position 65 of the light chain is modified to an arginine residue or an asparagine residue, the position 67 of the light chain is modified to an arginine residue or an asparagine residue, the position 70 of the light chain is modified to an arginine residue or an asparagine residue, and the position 72 of the light chain is modified to an arginine residue or an asparagine residue.

The anti-EGFR antibody contains an HCDR1 including the amino acid sequence of SEQ ID NO: 1, an HCDR2 including the amino acid sequence SGG, an HCDR3 including the amino acid sequence of SEQ ID NO: 2, an LCDR1 including the amino acid sequence of SEQ ID NO: 3, an LCDR2 including the amino acid sequence YAS, and an LCDR3 including the amino acid sequence of SEQ ID NO: 4. The sequence of each CDR is described below.
HCDR1: SEQ ID NO: 1
GFSLTNY
HCDR2:
SGG
HCDR3: SEQ ID NO: 2
LTYYDYEFA
LCDR1: SEQ ID NO: 3
SQSIGTN
LCDR2:
YAS
LCDR3: SEQ ID NO: 4
NNNWPT

Preferably, the amino acid sequence of the heavy chain of the anti-EGFR antibody includes the amino acid sequence of SEQ ID NO: 5.
SEQ ID NO: 5

QVQLKQSGPGLVQPSQSLSITCTVSGFSLTNYGVHWVRQSPGKGLEWLG
VIWSGGNTDYNTPFTSRLSINKDNSKSQVFFKMNSLQSNDTAIYYCARALTYYDY

EFAYWGQGTLVTVSAASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKVD
KKVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEYK
CKVSNKALPAPIEKTISKAKGQPREPQVYTLPPSRDELTKNQVSLTCLVKGFYPSD
IAVEWESNGQPENNYKTTPPVLDSDGSFFLYSKLTVDKSRWQQGNVFSCSVMHE
ALHNHYTQKSLSLSPGK

The heavy chain of the anti-EGFR antibody may be modified as long as ADCC activity (ADCC activity will be described later) is not lost from the anti-EGFR antibody.

The anti-EGFR antibody modified as described above has improved affinity for FcyRIIIa as compared to an unmodified anti-EGFR antibody. The "improvement in affinity" means that the affinity between the anti-EGFR antibody and FcyRIIIa is enhanced. The affinity between the anti-EGFR antibody and FcyRIIIa can be evaluated by a known method. For example, it can be confirmed by subjecting the anti-EGFR antibody to an affinity column using FcyRIIIa as a ligand and then measuring the elution time (retention time). An increase in the elution time of the anti-EGFR antibody subjected to an affinity column is an index of improvement in affinity. Examples of the affinity column include TSKgel (registered trademark) FcR-IIIA-NPR (manufactured by Tosoh Corporation). The affinity for FcyRIIIa may be evaluated by a kinetic parameter in a molecular interaction, or may be evaluated by an immunoassay such as an ELISA method. The kinetic parameter includes an association rate constant (kₒₙ), a dissociation rate constant (k_{off}), and a dissociation constant (K_{D}). For example, a decrease in dissociation constant is an indicator of an improvement in affinity. Examples of an index of affinity by immunological measurement include a 50% effective concentration (EC50). The kinetic parameter in a molecular interaction can be obtained by surface plasmon resonance (SPR) technology.

It is considered that the ADCC activity is improved by improving the affinity for FcyRIIIa. ADCC refers to a reaction in which an antibody bound to an antigen on the surface of a cell such as a target cell (for example, a tumor cell) activates an effector cell in vivo through binding between an Fc region of the antibody and an Fc receptor of an effector cell such as a natural killer cell or an eosinophil, thereby killing the target cell. It is considered that improving the affinity for FcyRIIIa can improve the ADCC activity, thereby improving the killing effect on a target cell (for example, a cancer cell). The ADCC activity can be evaluated using a known method, for example, by mixing and incubating an effector cell, a tumor cell, and an antibody, and measuring the number of killed tumor cell.

The light chain of the anti-EGFR antibody including the modification of amino acid residue includes, for example, the amino acid sequence of SEQ ID NO: 6, 7, or 8. SEQ ID NO: 6, a sequence of the light chain of the anti-EGFR antibody, is an amino acid sequence in which the amino acid residues at positions 65, 67, and 70 of the light chain are substituted with arginine residues as compared with an unmodified antibody. SEQ ID NO: 7, a sequence of the light chain of the anti-EGFR antibody, is an amino acid sequence in which the amino acid residues at positions 65, 67, 70, and 72 of the light chain are substituted with arginine residues as compared with an unmodified antibody. SEQ ID NO: 8, a sequence of the light chain of the anti-EGFR antibody, is an amino acid sequence in which the amino acid residues at positions 63, 65, 67, 70, and 72 of the light chain are substituted with aspartic acid residues as compared with an unmodified antibody. These amino acid sequences are shown below. The arginine residues and aspartic acid residues introduced by the modification are underlined.
SEQ ID NO: 6 
DILLTQSPVILSVSPGERVSFSCRASQSIGTNIHWYQQRTNGSPRLLIKYAS
ESISGIPSRFSGRGRGTRFTLSINSVESEDIADYYCQQNNNWPTTFGAGTKLELKRT
VAAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC
SEQ ID NO: 7 
DILLTQSPVILSVSPGERVSFSCRASQSIGTNIHWYQQRTNGSPRLLIKYAS
ESISGIPSRFSGRGRGTRFRLSINSVESEDIADYYCQQNNNWPTTFGAGTKLELKRT
VAAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC
SEQ ID NO: 8

DILLTQSPVILSVSPGERVSFSCRASQSIGTNIHWYQQRTNGSPRLLIKYAS
ESISGIPSRFDGDGDGTDFDLSINSVESEDIADYYCQQNNNWPTTFGAGTKLELKR
TVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC

The anti-EGFR antibody may be an antibody derived from any animal, and examples thereof include antibodies derived from human, mouse, rat, hamster, rabbit, goat, horse, chicken, and the like. The anti-EGFR antibody may be a chimeric antibody, a humanized antibody, or a human antibody. The chimeric antibody is suitably a human-non-human animal chimeric antibody, and more suitably a human-mouse chimeric antibody. The class of the anti-EGFR antibody may be any of IgG, IgA, IgM, IgD, and IgE, and is preferably IgG. In addition to the modification introduced for the purpose of improving the affinity for FcyRIIIa, the anti-EGFR antibody may have an amino acid residue modification for another purpose. Examples thereof include a modification for improving the affinity for the antigen, a modification for improving the affinity for FcRn, a modification for suppressing aggregation of the antibody, a modification for improving the solubility of the antibody, a modification for improving the thermal stability of the antibody, and the like. The anti-EGFR antibody may be an isolated antibody or a recombinant antibody.

The (modified) anti-EGFR antibody can be used for the same application for which an unmodified antibody is used. For example, it can be used as an active ingredient of an anticancer agent.

### (Method for producing anti-EGFR antibody)

The present invention also relates to a method for producing the anti-EGFR antibody (hereinafter, also simply referred to as a production method).

The production method includes a step of generating an anti-EGFR antibody and a step of collecting the anti-EGFR antibody. Specifically, a polynucleotide encoding an amino acid sequence of a (modified) anti-EGFR antibody is provided. Then, using the polynucleotide, a (modified) anti-EGFR antibody is generated and collected by a protein expression system. The protein expression system may be an expression system using a host cell or a cell-free protein synthesis system. Examples of the host cell include a mammalian cell, an insect cell, Escherichia coli, yeast, and the like. Examples of the cell-free protein synthesis system include a wheat germ derived synthesis system, an E. coli-derived synthesis system, a reconfigurable cell-free protein synthesis system, and the like.

An example of a method for producing a polynucleotide encoding the (modified) anti-EGFR antibody is as follows. First, with respect to an unmodified anti-EGFR antibody, modification of amino acid residue is introduced into the antibody by a known molecular biological technique such as a DNA recombination technique. Preferably, the unmodified anti-EGFR antibody does not comprise three residues which are an arginine or aspartic acid in residue positions 57, 60, 63, 65, 67, 70, 72, 74, 76, 77, and 79 to 81 of the light chain. For example, when there is a hybridoma that produces an unmodified anti-EGFR antibody, each of a polynucleotide encoding the light chain and a polynucleotide encoding the heavy chain is synthesized using an RNA extracted from the hybridoma by a reverse transcription reaction and a rapid amplification of cDNA ends (RACE) method. Among the polynucleotides, by amplifying the polynucleotide encoding the light chain by a PCR method using a primer for introducing a modification into at least three amino acid residues in the FR3 of the light chain, a polynucleotide encoding the light chain in which the modification has been introduced into the FR3 can be obtained.

An example of production of the anti-EGFR antibody according to the invention when a host cell is used is as follows. First, each of the polynucleotides is incorporated into an expression vector known in the art. The polynucleotide encoding the light chain and the polynucleotide encoding the heavy chain may be incorporated into one expression vector or may be separately incorporated into two expression vectors. The type of expression vector is not particularly limited, but may be an expression vector for a mammalian cell or an expression vector for E. coli. The obtained expression vector is introduced (transfected or transformed) into an appropriate host cell (for example, a mammalian cell or E. coli) and then expressed in the host cell, whereby the modified anti-EGFR antibody can be generated. Next, the host cell is dissolved in a solution containing an appropriate solubilizing agent to release the anti-EGFR antibody into the solution. When the host cell secretes the modified anti-EGFR antibody into the medium, the culture supernatant is collected.

Once a stable strain of the host cell into which the expression vector has been introduced is established, the antibody can be produced by culturing the cell under an appropriate condition as long as the cell doesn't die. In addition, the expression vector incorporating the polynucleotide encoding the antibody can be stored, and can be introduced into a host cell for antibody production and collection, as required. The expression vector can be stored by cryopreserving a solution containing a polynucleotide that is the expression vector or a cell containing the expression vector.

When the anti-EGFR antibody according to the invention is obtained by the cell-free protein synthesis system, a polynucleotide encoding the light chain in which FR3 has been modified and a polynucleotide encoding the heavy chain (optionally of an unmodified anti-EGFR antibody) are added to the cell-free protein synthesis system, followed by incubation under an appropriate condition, whereby the modified antibody can be produced and collected.

Collection of the antibody can be performed by a known method such as affinity chromatography. As required, the collected antibody may be purified by a known method such as gel filtration.

### (Method for improving affinity of anti-EGFR antibody for FcyRIIIa)

The present invention also relates to a method for improving the affinity of an anti-EGFR antibody for FcyRIIIa. By modifying at least three amino acid residues in the FR3 to arginine residues or aspartic acid residues in an unmodified anti-EGFR antibody, the affinity of the anti-EGFR antibody for FcyRIIIa can be improved. The (modified) anti-EGFR antibody of the invention and the unmodified anti-EGFR antibody are as described above.

Whether or not the affinity of the anti-EGFR antibody for FcyRIIIa is improved can be confirmed, for example, by subjecting the anti-EGFR antibody to an affinity column using FcyRIIIa as a ligand and then measuring the elution time. Alternatively, it can also be evaluated by a kinetic parameter in a molecular interaction or an immunoassay such as an ELISA method. The kinetic parameter includes an association rate constant (kₒₙ), a dissociation rate constant (k_{off}), and a dissociation constant (K_{D}), and K_{D} is preferable. Examples of an index of affinity by immunological measurement include a 50% effective concentration (EC50). Alternatively, it can also be evaluated by measuring the ADCC activity of the anti-EGFR antibody for FcyRIIIa.

When the affinity of the anti-EGFR antibody for FcyRIIIa is improved, the value of K_{D} in the antigen-antibody reaction is, for example, about 1/2, about 1/5, about 1/10, about 1/20, about 1/50, about 1/100, or about 1/1000 compared to an unmodified antibody.

### (Method for improving ADCC activity of anti-EGFR antibody)

The present invention further relates to a method for improving the ADCC activity of an anti-EGFR antibody in the anti-EGFR antibody containing an HCDR1 including the amino acid sequence of SEQ ID NO: 1, an HCDR2 including the amino acid sequence of SGG, an HCDR3 including the amino acid sequence of SEQ ID NO: 2, an LCDR1 including the amino acid sequence of SEQ ID NO: 3, an LCDR2 including the amino acid sequence of YAS, and an LCDR3 including the amino acid sequence of SEQ ID NO: 4, by modifying at least three amino acid residues in the FR3 to arginine residues or aspartic acid residues. By modifying at least three amino acid residues in the FR3 to arginine residues or aspartic acid residues in an unmodified anti-EGFR antibody, the affinity of the anti-EGFR antibody for FcyRIIIa is improved. Thus, the ADCC activity of the anti-EGFR antibody can be improved. The (modified) anti-EGFR antibody of the invention is as described above.

### (Polynucleotide)

The present invention further also relates to a polynucleotide encoding the (modified) anti-EGFR antibody according to the invention. The polynucleotide may be in a state of being incorporated into an appropriate vector.

### (Host cell)

The present invention further also relates to a host cell containing the vector. The type of the host cell is not particularly limited, but may be a eukaryotic cell, a prokaryotic cell, or the like. The eukaryotic cell is preferably an animal cell, and more preferably a mammalian cell.

### (Pharmaceutical composition)

The present invention further also relates to a pharmaceutical composition containing the modified anti-EGFR antibody. The pharmaceutical composition may contain a component other than the modified anti-EGFR antibody. The pharmaceutical composition preferably contains the anti-EGFR antibody in a pharmacologically effective amount.

The pharmaceutical composition may contain a pharmaceutically acceptable additive. Such an additive can be appropriately selected from additives known in the art. Examples of the known additives include an excipient, a lubricant, a binder, a disintegrant, a coating agent, a capsule base, a plasticizer, a colorant, a solvent, a stabilizer, a preservative, a buffer, a soothing agent, a base, an emulsifier, a suspending agent, a flavoring agent, a sweetening agent, an adsorbent, a solubilizing agent, a pH adjuster, a thickener, an isotonizing agent, a dispersant, a preservative, a wetting agent, a flavoring agent, an antioxidant, and the like.

The formulation of the pharmaceutical composition is not particularly limited, but may be any formulation known to those skilled in the art, such as a solid preparation, a semi-solid preparation, a liquid preparation, an injection, or a suppository. Specific examples of the dosage form include, but are not limited to, an injection and a solution.

The (modified) anti-EGFR antibody of the invention can be suitably used for treatment of various tumors. The tumor is not particularly limited, but examples thereof include large bowel cancer, colon cancer, rectal cancer, head and neck cancer, pharyngeal cancer, oral cancer, laryngeal cancer, pancreatic cancer, lung cancer, and breast cancer.

Hereinafter, a detailed description is made of the present invention with reference to Examples, but the present invention is not limited to the Examples.

### EXAMPLES

### Example: Acquisition of anti-EGFR antibody and measurement of FcyRIIIa binding affinity

An FR3-modified anti-EGFR antibody was generated, and FcyRIIIa binding affinity was measured.

### (Acquisition of anti-EGFR antibody)

The FR3 of the light chain of an anti-EGFR antibody was modified to give three modified antibodies (hereinafter, modified antibodies are individually referred to as R3, R4, and D5). Specifically, this was performed as follows.

Polynucleotides encoding the amino acid sequences of SEQ ID NOs: 6, 7, and 8 were prepared. Each polynucleotide was incorporated into a plasmid, and the plasmid was transfected into an Expi293 cell. A culture supernatant of the cell expressing an anti-EGFR modified antibody was collected, and the anti-EGFR modified antibody expressed was purified from the culture supernatant to give each anti-EGFR modified antibody.

These modified antibodies included an amino acid residue modification in the FR3 of the light chain as compared to the FR3 in the light chain of an unmodified anti-EGFR antibody. Specifically, the R3 (SEQ ID NO: 6) included modifications to arginine residues at positions 65, 67, and 70 of the light chain, the R4 (SEQ ID NO: 7) included modifications to arginine residues at positions 65, 67, 70, and 72 of the light chain, and the D5 (SEQ ID NO: 8) included modifications to aspartic acid residues at positions 63, 65, 67, 70, and 72 of the light chain. All modifications of these amino acid residues were substitutions.
R3 light chain: SEQ ID NO: 6 
DILLTQSPVILSVSPGERVSFSCRASQSIGTNIHWYQQRTNGSPRLLIKYAS
ESISGIPSRFSGRGRGTRFTLSINSVESEDIADYYCQQNNNWPTTFGAGTKLELKRT
 
VAAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC
R4 light chain: SEQ ID NO: 7 
DILLTQSPVILSVSPGERVSFSCRASQSIGTNIHWYQQRTNGSPRLLIKYAS
ESISGIPSRFSGRGRGTRFRLSINSVESEDIADYYCQQNNNWPTTFGAGTKLELKRT
VAAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC
D5 light chain: SEQ ID NO: 8

DILLTQSPVILSVSPGERVSFSCRASQSIGTNIHWYQQRTNGSPRLLIKYAS
ESISGIPSRFDGDGDGTDFDLSINSVESEDIADYYCQQNNNWPTTFGAGTKLELKR
TVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC

The amino acid sequences of the HCDR1, HCDR2, HCDR3, LCDR1, LCDR2, and LCDR3, and heavy chain of these modified antibodies were as follows.
HCDR1: SEQ ID NO: 1
GFSLTNY
HCDR2:
SGG
HCDR3: SEQ ID NO: 2
LTYYDYEFA
LCDR1: SEQ ID NO: 3
SQSIGTN
LCDR2:
YAS
LCDR3: SEQ ID NO: 4
NNNWPT
Heavy Chain: SEQ ID NO: 5

QVQLKQSGPGLVQPSQSLSITCTVSGFSLTNYGVHWVRQSPGKGLEWLG
VIWSGGNTDYNTPFTSRLSINKDNSKSQVFFKMNSLQSNDTAIYYCARALTYYDY
EFAYWGQGTLVTVSAASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKVD
KKVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEYK
CKVSNKALPAPIEKTISKAKGQPREPQVYTLPPSRDELTKNQVSLTCLVKGFYPSD
IAVEWESNGQPENNYKTTPPVLDSDGSFFLYSKLTVDKSRWQQGNVFSCSVMHE
ALHNHYTQKSLSLSPGK

The sequence of the light chain of an unmodified anti-EGFR antibody was as shown in SEQ ID NO: 9.
Light chain of unmodified anti-EGFR antibody: SEQ ID NO: 9

DILLTQSPVILSVSPGERVSFSCRASQSIGTNIHWYQQRTNGSPRLLIKYAS

ESISGIPSRFSGSGSGTDFTLSINSVESEDIADYYCQQNNNWPTTFGAGTKLELKRT
VAAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC

The FcyRIIIa binding affinity of these modified and unmodified anti-EGFR antibodies was confirmed. For confirmation of FcyRIIIa binding affinity, TSKgel (registered trademark) FcR-IIIA-NPR (manufactured by Tosoh Corporation) which was an affinity chromatography column was used. This column was a column capable of separating an antibody based on the binding affinity of the antibody for FcyRIIIa. The analysis condition was as follows.

**[Table 1]**

| | |
|---|---|
| Analyzer | High performance liquid chromatograph Prominence (manufactured by Shimazu Corporation) |
| Detector | Ultraviolet absorptiometer |
| Analysis condition | Measure wavelength at 280 nm |
| Column | TSKgel (registered trademark) FcR-IIIA-NPR (4.6 mm I.D. × 7.5 cm) |
| Temperature | **25°C** |
| Flow rate | **1.0 mL** / **min** |
| Sample | 5 µg of purified antibody |
| Eluate A | 50 mM citrate buffer (pH 6.5) |
| Eluate B | 50 mM citrate buffer (pH 4.5) |

**[Table 2]**

| Gradient condition | | | |
|---|---|---|---|
| Time after injection (min) | Eluate A (vol %) | Eluate B (vol %) | Note |
| **0-2** | **100** | **0** | |
| **2-20** | **100-0** | **0-100** | Linear gradient |

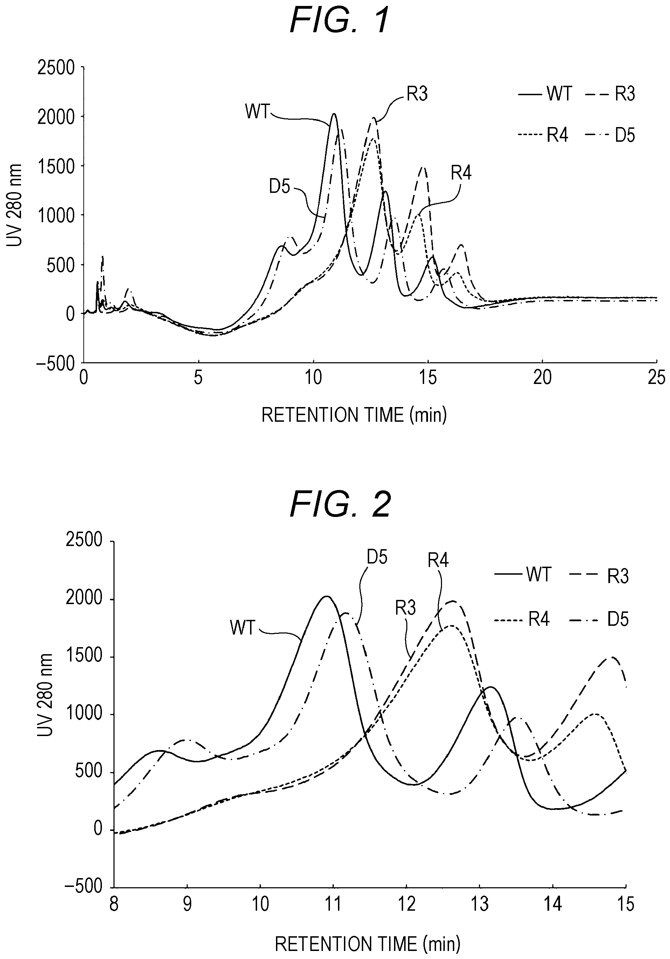

Each anti-EGFR modified antibody was applied to the column under the condition described above, and the elution time of the antibody from the column was measured. The results are shown in Fig. 1. In Fig. 1, the vertical axis represents absorbance at 280 nm, and the horizontal axis represents elution time. Fig. 2 is an enlarged view of Fig. 1, including the strongest peak portion of each graph. In the figures, "WT" represents an unmodified antibody. From Figs. 1 and 2, it has been found that the elution time was delayed, because the peaks of the elution time of the modified antibodies were shifted to the right side as compared with the peak of the elution time of the unmodified antibody. Introducing modification of an arginine residue or an aspartic acid residue into the light chain of FR3 could improve the affinity of the anti-EGFR antibody for FcyRIIIa.

### [Sequence listing]

### [Item 1]

A method for producing an anti-EGFR antibody, comprising: generating an anti-EGFR antibody having an amino acid sequence of the anti-EGFR antibody using a polynucleotide encoding the amino acid sequence; and collecting the anti-EGFR antibody, wherein the anti-EGFR antibody contains an HCDR1 including an amino acid sequence of SEQ ID NO: 1, an HCDR2 including an amino acid sequence SGG, an HCDR3 including an amino acid sequence of SEQ ID NO: 2, an LCDR1 including an amino acid sequence of SEQ ID NO: 3, an LCDR2 including an amino acid sequence YAS, and an LCDR3 including an amino acid sequence of SEQ ID NO: 4, the anti-EGFR antibody contains an amino acid sequence in which at least three amino acid residues in a framework region 3 of a light chain have been modified to arginine residues or aspartic acid residues, and the HCDR1, HCDR2, HCDR3, LCDR1, LCDR2, LCDR3, and framework region 3 are defined by a Chothia method.

### [Item 2]

A method for improving affinity of an anti-EGFR antibody for FcyRIIIa as compared with affinity before modification, comprising modifying at least three amino acid residues in a framework region 3 of a light chain to arginine residues or aspartic acid residues in the anti-EGFR antibody containing an HCDR1 including an amino acid sequence of SEQ ID NO: 1, an HCDR2 including an amino acid sequence SGG, an HCDR3 including an amino acid sequence of SEQ ID NO: 2, an LCDR1 including an amino acid sequence of SEQ ID NO: 3, an LCDR2 including an amino acid sequence YAS, and an LCDR3 including an amino acid sequence of SEQ ID NO: 4, wherein the HCDR1, HCDR2, HCDR3, LCDR1, LCDR2, LCDR3, and framework region 3 are defined by a Chothia method.

### [Item 3]

The method according to item 1 or 2, wherein the at least three amino acid residues are at least three amino acid residues selected from the group consisting of amino acid residues at positions 63, 65, 67, 70, and 72 of the light chain.

### [Item 4]

The method according to item 1 or 2, wherein the at least three amino acid residues include amino acid residues at positions 65, 67 and 70 of the light chain.

### [Item 5]

The method according to item 1 or 2, wherein the at least three amino acid residues include amino acid residues at positions 65, 67, 70, and 72 of the light chain.

### [Item 6]

The method according to item 1 or 2, wherein the at least three amino acid residues include amino acid residues at positions 63, 65, 67, 70, and 72 of the light chain.

### [Item 7]

The method according to item 1 or 2, wherein a heavy chain of the anti-EGFR antibody has an amino acid sequence of SEQ ID NO: 5.

### [Item 8]

The method according to item 1 or 2, wherein the light chain of the anti-EGFR antibody comprises an amino acid sequence of SEQ ID NO: 6, an amino acid sequence of SEQ ID NO: 7, or an amino acid sequence of SEQ ID NO: 8.

### [Item 9]

The method according to item 1 or 2, wherein the at least three amino acid residues are 3 or more and 5 or less amino acid residues.

### [Item 10]

An anti-EGFR antibody, comprising an HCDR1 including an amino acid sequence of SEQ ID NO: 1; an HCDR2 including an amino acid sequence SGG; an HCDR3 including an amino acid sequence of SEQ ID NO: 2; an LCDR1 including an amino acid sequence of SEQ ID NO: 3; an LCDR2 including an amino acid sequence YAS; and an LCDR3 including an amino acid sequence of SEQ ID NO: 4, wherein at least three amino acid residues in a framework region 3 of a light chain have been modified to arginine residues or aspartic acid residues, and the HCDR1, HCDR2, HCDR3, LCDR1, LCDR2, LCDR3, and framework region 3 are defined by a Chothia method.

### [Item 11]

The antibody according to item 10, wherein the at least three amino acid residues are at least three amino acid residues selected from the group consisting of amino acid residues at positions 63, 65, 67, 70, and 72 of the light chain.

### [Item 12]

The antibody according to item 10, wherein the at least three amino acid residues include amino acid residues at positions 65, 67 and 70 of the light chain.

### [Item 13]

The antibody according to item 10, wherein the at least three amino acid residues include amino acid residues at positions 65, 67, 70, and 72 of the light chain.

### [Item 14]

The antibody according to item 10, wherein the at least three amino acid residues include amino acid residues at positions 63, 65, 67, 70, and 72 of the light chain.

### [Item 15]

The antibody according to item 10, wherein a heavy chain has an amino acid sequence of SEQ ID NO: 5.

### [Item 16]

The antibody according to item 10, wherein the light chain comprises an amino acid sequence of SEQ ID NO: 6, an amino acid sequence of SEQ ID NO: 7, or an amino acid sequence of SEQ ID NO: 8.

### [Item 17]

The antibody according to item 10, wherein the at least three amino acid residues are 3 or more and 5 or less amino acid residues.

## Claims

1. A method for producing an anti-EGFR antibody, comprising: generating an anti-EGFR antibody having an amino acid sequence of the anti-EGFR antibody using a polynucleotide encoding the amino acid sequence; and collecting the anti-EGFR antibody,
wherein the anti-EGFR antibody comprises: an HCDR1 comprising an amino acid sequence of SEQ ID NO: 1, an HCDR2 comprising an amino acid sequence SGG, an HCDR3 comprising an amino acid sequence of SEQ ID NO: 2, an LCDR1 comprising an amino acid sequence of SEQ ID NO: 3, an LCDR2 comprising an amino acid sequence YAS, and an LCDR3 comprising an amino acid sequence of SEQ ID NO: 4,
wherein the anti-EGFR antibody comprises an amino acid sequence in which at least three amino acid residues in a framework region 3 of a light chain have been modified each independently to an arginine residue or aspartic acid residue, and
wherein the HCDR1, HCDR2, HCDR3, LCDR1, LCDR2, LCDR3, and the framework region 3 are defined by the Chothia method.

2. A method for improving affinity of an anti-EGFR antibody for FcyRIIIa as compared with affinity before modification, comprising modifying at least three amino acid residues in a framework region 3 of a light chain each independently from a residue which is other than an arginine or aspartic acid residue to an arginine residue or aspartic acid residue in the anti-EGFR antibody,
wherein the anti-EGFR antibody comprises an HCDR1 comprising an amino acid sequence of SEQ ID NO: 1, an HCDR2 comprising an amino acid sequence SGG, an HCDR3 comprising an amino acid sequence of SEQ ID NO: 2, an LCDR1 comprising an amino acid sequence of SEQ ID NO: 3, an LCDR2 comprising an amino acid sequence YAS, and an LCDR3 comprising an amino acid sequence of SEQ ID NO: 4, and
the HCDR1, HCDR2, HCDR3, LCDR1, LCDR2, LCDR3, and the framework region 3 are defined by a Chothia method.

3. The method according to claim 1 or 2, wherein the at least three amino acid residues are at least three amino acid residues selected from the group consisting of amino acid residues at positions 63, 65, 67, 70, and 72 of the light chain, preferably wherein the amino acid residues are modified to arginine residues.

4. The method according to claim 1 or 2, wherein the at least three amino acid residues comprise at least amino acid residues at positions 65, 67 and 70 of the light chain, preferably wherein the amino acid residues are modified to arginine residues.

5. The method according to claim 1 or 2, wherein the at least three amino acid residues comprise at least the amino acid residues at positions 65, 67, 70, and 72 of the light chain, preferably wherein the amino acid residues are modified to arginine residues.

6. The method according to claim 1 or 2, wherein the at least three amino acid residues comprise at least the amino acid residues at positions 63, 65, 67, 70, and 72 of the light chain, preferably wherein the amino acid residues are modified to arginine residues.

7. The method according to claim 1 or 2, wherein a heavy chain of the anti-EGFR antibody comprise an amino acid sequence of SEQ ID NO: 5 and/or wherein the light chain of the anti-EGFR antibody comprises an amino acid sequence of SEQ ID NO: 6, an amino acid sequence of SEQ ID NO: 7, or an amino acid sequence of SEQ ID NO: 8.

8. The method according to claim 1 or 2, wherein the at least three amino acid residues are 3 or more and 5 or less amino acid residues.

9. An anti-EGFR antibody, comprising an HCDR1 comprising an amino acid sequence of SEQ ID NO: 1; an HCDR2 comprising an amino acid sequence SGG; an HCDR3 comprising an amino acid sequence of SEQ ID NO: 2; an LCDR1 comprising an amino acid sequence of SEQ ID NO: 3; an LCDR2 comprising an amino acid sequence YAS; and an LCDR3 comprising an amino acid sequence of SEQ ID NO: 4,
wherein at least three amino acid residues in a framework region 3 of a light chain have been modified each independently to an arginine residue or aspartic acid residue, and
the HCDR1, HCDR2, HCDR3, LCDR1, LCDR2, LCDR3, and the framework region 3 are defined by a Chothia method.

10. The antibody according to claim 9, wherein the at least three amino acid residues are at least three amino acid residues selected from the group consisting of amino acid residues at positions 63, 65, 67, 70, and 72 of the light chain.

11. The antibody according to claim 9 or 10, wherein the at least three amino acid residues comprise at least amino acid residues at positions 65, 67 and 70 of the light chain.

12. The antibody according to claim 9 or 10, wherein the at least three amino acid residues comprise at least amino acid residues at positions 65, 67, 70, and 72 of the light chain.

13. The antibody according to claim 9 or 10, wherein the at least three amino acid residues comprise at least amino acid residues at positions 63, 65, 67, 70, and 72 of the light chain.

14. The antibody according to claim 10 or 11, wherein a heavy chain has an amino acid sequence of SEQ ID NO: 5; and/or wherein the light chain comprises an amino acid sequence of SEQ ID NO: 6, an amino acid sequence of SEQ ID NO: 7, or an amino acid sequence of SEQ ID NO: 8.

15. The antibody according to claim 10 or 11, wherein the at least three amino acid residues are 3 or more and 5 or less amino acid residues.
